# EUROPEAN PATENT SPECIFICATION

(11) **EP 3 515 347 B1**
(45) Date of publication and mention of the grant of the patent: **12.02.2020**
(21) Application number: 17769045.0
(22) Date of filing: 18.09.2017
(51) Int. Cl.: A61B 18/20, A61B 18/00

(54) **A CUTTING ASSEMBLY FOR A HAIR CUTTING DEVICE**
SCHNEIDEANORDNUNG FÜR EINE HAARSCHNEIDEVORRICHTUNG
ENSEMBLE DE COUPE POUR DISPOSITIF POUR COUPER LES CHEVEUX

(30) Priority: 21.09.2016 EP 16189909
(43) Date of publication of application: 31.07.2019
(73) Proprietor: Koninklijke Philips N.V., 5656 AE Eindhoven (NL)
(72) Inventor: THUMMA, Kiran, Kumar, 5656 AE Eindhoven (NL); VERHAGEN, Rieko, 5656 AE Eindhoven (NL); JOHNSON, Mark, Thomas, 5656 AE Eindhoven (NL); MOESKOPS, Bastiaan, Wilhelmus, Maria, 5656 AE Eindhoven (NL); BOAMFA, Marius, Iosif, 5656 AE Eindhoven (NL)
(74) Representative: Uittenbroek, Arie Leendert
(86) International application number: PCT/EP2017/073389
(87) International publication number: WO 2018/054800

(56) References cited:
- WO-A1-2014/143670
- US-A- 5 272 330
- US-A1- 2008 244 912
- US-A1- 2012 123 444
- US-B1- 6 631 234

## Description

### FIELD OF THE INVENTION

The invention relates to a cutting assembly for use with a hair cutting device for cutting (e.g. shaving) hair on a body of a subject and, in particular, a cutting assembly having a tapered portion. The invention also relates to a hair cutting device comprising such a cutting assembly.

### BACKGROUND OF THE INVENTION

Shaving devices for cutting or shaving hair on a body of a subject typically make use of one or more blades that cut hairs as the device is moved across the skin of the subject. The blades can be static within the device, for example as in a wet razor, whereas in other types of devices, for example electric shavers, one or more blade elements can be actuated (e.g. rotated or oscillated) in order to produce a cutting action.

However, an alternative type of shaving device has been proposed in WO 2014/143670 that makes use of laser light. In particular, a laser light source is provided that is configured to generate laser light having a wavelength selected to target a predetermined chromophore to effectively cut a hair shaft. A fibre optic is located on a shaving portion of the device that is positioned to receive the laser light from the laser light source at a proximal end, conduct the laser light from the proximal end toward a distal end, and emit the light out of a cutting region of the fibre optic and toward hair when the cutting region is brought in contact with the hair.

### SUMMARY OF THE INVENTION

In order to cut or melt hair, sufficient optical energy needs to be delivered through a cutting element of the cutting device. As light propagates through the cutting element, the intensity of the light may reduce, thereby reducing the efficiency of the cutting element away from the point of entry of light into the cutting element.

Thus, there exists a need for a cutting assembly having a cutting element with an increased active area and/or with a more consistent efficiency over its length.

According to a first aspect, there is provided a cutting assembly for use in a hair cutting device, the cutting assembly comprising a cutting element having a first end and a second end, the cutting element comprising an optical waveguide for receiving light from at least one light source; a first light guiding element configured to guide light from the at least one light source into the first end of the cutting element; and a second light guiding element configured to guide light from the at least one light source into the second end of the cutting element. Each of the light guiding elements comprises a taper transition section in which a diameter of the light guiding element reduces from a first diameter to a second diameter. By guiding light into both ends of the cutting element, the intensity of the light propagating through the cutting element is high at both ends. Thus, compared to a cutting element having light guided into a single end thereof, the cutting element of the cutting assembly of the present invention provides a larger active cutting area and, therefore, a more effective hair cutting device.

In some embodiments, the cutting assembly may further comprise a beam splitter for dividing light from the at least one light source into two paths (i.e. optical paths), a first path along the first light guiding element from the beam splitter to the first end of the cutting element, and a second path along the second light guiding element from the beam splitter to the second end of the cutting element. The beam splitter may comprise a 50-50 coupler. By incorporating a beam splitter into the cutting assembly, or into the hair cutting device, the desired effect (i.e. light entering the cutting element from both ends) can be achieved using a single light source.

The first light guiding element may be configured to guide light from a first light source to the first end of the cutting element. The second light guiding element may be configured to guide light from a second light source to the second end of the cutting element. By using multiple light sources, light having different wavelengths may be guided into the cutting element.

According to some embodiments, the first light guiding element and/or the second light guiding element may comprise a portion of the optical waveguide. At least one of the first light guiding element, the second light guiding element and the cutting element may comprise an optical fibre. Such arrangements may be constructed from a single optical waveguide, such as a single optical fibre.

According to a second aspect, there is provided a hair cutting device for cutting hair on a body of a subject, the hair cutting device comprising at least one light source for generating laser light at one or more specific wavelengths corresponding to wavelengths absorbed by one or more chromophores in hair; and a cutting assembly coupled to the at least one light source. The cutting assembly comprises a cutting element having a first end and a second end, the cutting element comprising an optical waveguide for receiving light from the at least one light source; a first light guiding element configured to guide light from the at least one light source into the first end of the cutting element; and a second light guiding element configured to guide light from the at least one light source into the second end of the cutting element. Each of the light guiding elements comprises a taper transition section in which a diameter of the light guiding element reduces from a first diameter to a second diameter.

In some embodiments, the hair cutting device or the cutting assembly may further comprise a beam splitter for dividing light from the at least one light source into two paths, a first path along the first light guiding element from the beam splitter to the first end of the cutting element, and a second path along the second light guiding element from the beam splitter to the second end of the cutting element. The beam splitter comprises a 50-50 coupler.

In some embodiments, the at least one light source may comprise a single light source. In other embodiments, the at least one light source may comprise a first light source and a second light source. Light from the first light source may be guided along the first light guiding element to the first end of the cutting element, and light from the second light source may be guided along the second light guiding element to the second end of the cutting element.

According to some embodiments, the first light guiding element and/or the second light guiding element of the hair cutting device may comprise a portion of the optical waveguide. At least one of the first light guiding element, the second light guiding element and the cutting element may comprise an optical fibre.

Other advantageous features will become apparent from the following description.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a block diagram of a hair cutting device according to embodiments of the invention;
Fig. 2 is a pair of schematic drawings showing different views of an exemplary hair cutting device according to embodiments of the invention;
Fig. 3 is a graph illustrating the refractive index of hair;
Fig. 4 is an illustration of a portion of a hair cutting device according to embodiments of the invention; and
Fig. 5 is an illustration of a portion of a hair cutting device according to alternative embodiments of the invention.

### DETAILED DESCRIPTION OF THE EMBODIMENTS

As noted above, the present invention provides an improvement in the cutting ability and efficiency of a laser light-based shaving device, for example as described in WO 2014/143670. In particular, it has been recognised that cutting efficiency can be improved by introducing a tapered portion (i.e. a portion of reduced diameter) in the cutting element of an optical waveguide. By improving the consistency of the intensity of light over the cutting element, the cutting element has a larger active area to be used for cutting hair, resulting in a more rapid and efficient hair cutting experience. Consequently, the need for a user to repeatedly use the shaving device over the same area of his or her skin is reduced, along with the risk of pain or irritation of the skin.

It will be appreciated that the invention is applicable to shaving devices (e.g. razors or electric shavers), and any other type of device that is used to cut hair (e.g. hair clippers), even if those devices do not necessary aim to provide a 'clean shave' (i.e. to remove hair at the level of the skin).

As discussed below, the cutting element may, in some examples, include an optical waveguide, such as an optical fibre. Light propagating through the optical fibre may couple out of the fibre into a surrounding medium, forming an evanescent field. When a hair is brought into contact with or placed in very close proximity to the fibre, it may interact with the evanescent field surrounding the fibre and thereby cause light being transmitted from the fibre to the hair. This interaction may initiate cutting or melting of the hair. In order to ensure effective cutting, the interaction of the hair with the light field around the fibre should be such that significant out-coupling is ensured. It has been discovered that providing a tapered portion (i.e. a portion having a reduced diameter) in the optical fibre improves the cutting element efficiency. Using tapering, a numerical aperture (NA) of the guided beam is increased and the tapering ratio can be chosen such that it is close or equal to the maximum NA supported by the fibre, effectively maximizing the extent of the evanescent field and therewith the interaction depth of the evanescent field within the hair.

The inventors realized that using a tapered portion to increase the NA of the beam in the fibre might provide penetration depths which otherwise could only have been achieved by using much more powerful laser sources. By allowing less powerful laser sources to be used in the hair cutting device not only improves the energy efficiency of the hair cutting device, but also contributes to the overall safety of the hair cutting device.

The inventors realized that a further effect of increasing the NA of the light beam through the fibre increases the intensity incident on an object touching the fibre, such as a hair that is placed in contact with the fibre, in itself. A light ray travelling with a higher NA will have more interactions with the edge of the fibre per unit of length compared to a light particle travelling with a lower NA through the same fibre. As an example, assuming that the hair is a relatively large object compared to the thickness of the light-guide, it can be noted that a single light-ray has a certain opportunity to interact with the hair whereby the likelihood that it does is larger for the high NA light because it will strike the fibre surface at a higher rate, effectively increasing the probability for interaction (in other words, the absorption cross-section of the hair is effectively increased).

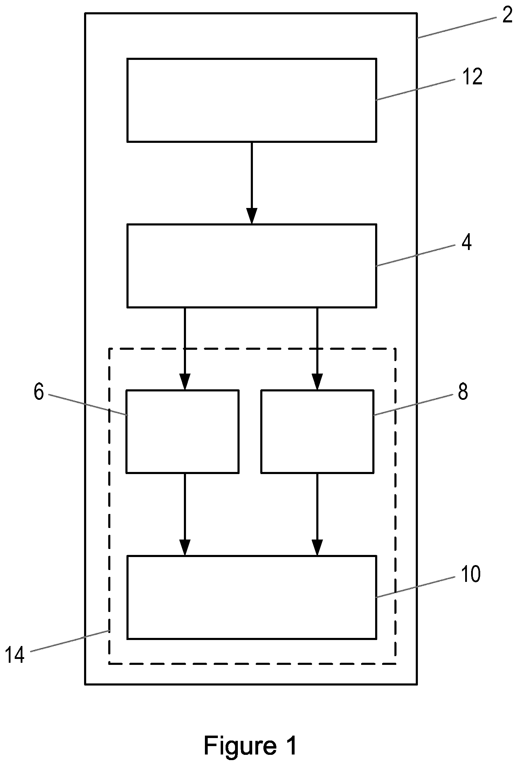

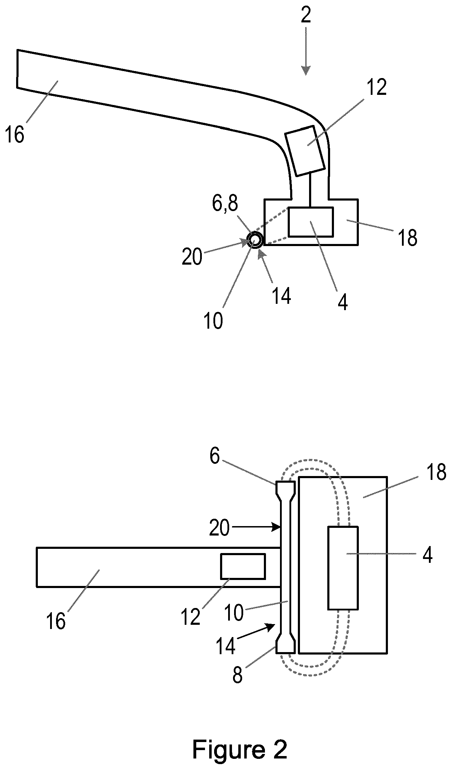

Fig. 1 is a block diagram of a hair cutting device 2 according to an embodiment of the invention. Fig. 2 shows a hair cutting device 2 in the form of a handheld razor according to an exemplary embodiment of the invention. The hair cutting device 2 is for cutting (e.g. shaving) hair on a body of a subject. The subject may be a person or an animal. The hair may be facial hair (i.e. hair on the subject's face), or hair on the subject's head or other part of their body (legs, chest, etc.).

Referring to Fig. 1, the hair cutting device 2 comprises at least one light source 4, such as a laser light source, for generating light at one or more specific wavelengths corresponding to wavelengths absorbed by one or more chromophores in hair. As discussed below, the at least one light source 4 may comprise a single light source or multiple light sources. The light emitted by the at least one light source 4 is coupled into a first light guiding element 6 and a second light guiding element 8 which guide the light towards a cutting element 10. The cutting element 10 enables hair to be cut as the hair cutting device 2 is moved over the skin of a subject. The cutting element 10 is an optical waveguide 10 that is arranged on the hair cutting device 2 so that the optical axis of the optical waveguide 10 (i.e. the line along which light typically propagates through the optical waveguide 10) is generally perpendicular to the direction in which the hair cutting device 2 is moved so that hairs contact the sidewall of the optical waveguide 10 (the sidewall corresponding to the long edge of the optical waveguide 10) as the hair cutting device 2 is moved across the skin of the subject. In some embodiments, the optical waveguide 10 is an optical fibre, although those skilled in the art will be aware of other types of optical waveguide that can be used according to the invention, such as a slab waveguide, a strip waveguide or a photonic crystal waveguide. An optical fibre comprises a core, and in some embodiments also comprises a cladding, which may or may not fully encompass the core (e.g. part of the core may be exposed).

The light (which in some embodiments may be laser light) that is generated by the at least one light source 4 is coupled into the optical waveguide 10 (and specifically coupled into both ends of the optical waveguide 10 so that the laser light propagates through the optical waveguide 10 from both ends).

The at least one light source 4 is configured to generate laser light at one or more specific wavelengths that can be used to cut or burn through hair. In particular, each wavelength corresponds to the wavelength of light absorbed by a chromophore that is found in hair. As is known, a chromophore is the part of a molecule that provides the molecule with its colour. Thus, the laser light will be absorbed by the chromophore and converted into heat which will melt or burn the hair or otherwise destroy the bonds in the molecules of the hair, and it is this melting or burning that provides the cutting action of the hair cutting device 2.

Suitable chromophores that can be targeted by the laser light generated by the at least one light source 4 include, but are not limited to, melanin, keratin and water. Suitable wavelengths of laser light that can be used include, but are not limited to, wavelengths selected from the range 380 nm (nanometres) to 500 nm and 2500 nm to 3500 nm. Those skilled in the art will be aware of the wavelengths of light that are absorbed by these chromophores, and thus also the specific wavelengths of light that the at least one light source 4 should generate for this purpose, and further details are not provided herein.

In some embodiments the at least one light source 4 can be configured to generate laser light at a plurality of wavelengths (either simultaneously or sequentially), with each wavelength being selected to target a different type of chromophore. This can improve the cutting action of the optical waveguide 4 since multiple types of molecules in the hair may be burnt using the laser light. In embodiments which include multiple light sources 4, each light source may generate laser light at a respective wavelength, and each light source can be coupled to a single optical waveguide 10 or to a respective optical waveguide to provide multiple cutting elements 10 in the device 2.

In some embodiments, the hair cutting device 2 also comprises a control unit 12 that controls the operation of the hair cutting device 2, and in particular is connected to the at least one light source 4 to control the activation and deactivation of the at least one light source 4 (and in some embodiments control the wavelength and/or intensity of the light generated by the at least one light source 4). The control unit 12 may activate and deactivate the at least one light source 4 in response to an input from a user of the hair cutting device 2. The control unit 12 can comprise one or more processors, processing units, multi-core processors or modules that are configured or programmed to control the hair cutting device 2.

The optical waveguide 10 (i.e. the cutting element) and the first and second light guiding elements 6, 8 may be considered to form components of a cutting assembly 14 which, in some embodiments, may be detachable from the hair cutting device 2, and may be enclosed in a separate unit or housing.

As noted above, Fig. 2 shows a hair cutting device 2 that is in the form of a handheld wet razor. Fig. 2 shows a side view and a bottom view of the razor 2. The razor 2 comprises a handle 16 for the subject (or other user of the device 2) to hold, and a head portion 18 that includes the cutting element 10 (optical waveguide/fibre). As shown, the optical waveguide 10 is arranged along an edge of the head portion, and a part of the optical waveguide 10 forms (or corresponds to) a cutting face 20. The cutting face 20 is the part of the optical waveguide 10 that is intended to come into contact with hair as the hair cutting device 2 is moved across the skin of the subject. Light from the at least one light source 4 is directed or guided into the first end of the optical waveguide 10 via the first light guiding element 6, and into the second end of the optical waveguide 10 via the second light guiding element 8. The at least one light source 4 and the control unit 12 are shown as being incorporated into the head portion 18 and handle 16 respectively, but it will be appreciated that the positions of these components in the hair cutting device 2 as shown in Fig. 2 is not limiting. Likewise it will be appreciated that the embodiment shown in Fig. 2 is merely an example, and the invention can be incorporated or used in any type of hair cutting device 2 that comprises an optical waveguide cutting element 10 as described herein. As noted above, the optical waveguide 10, the first light guiding element 6 and the second light guiding element 8 may form part of a cutting assembly 14 which may itself form a part of, or be detachably connected to, the head portion 18.

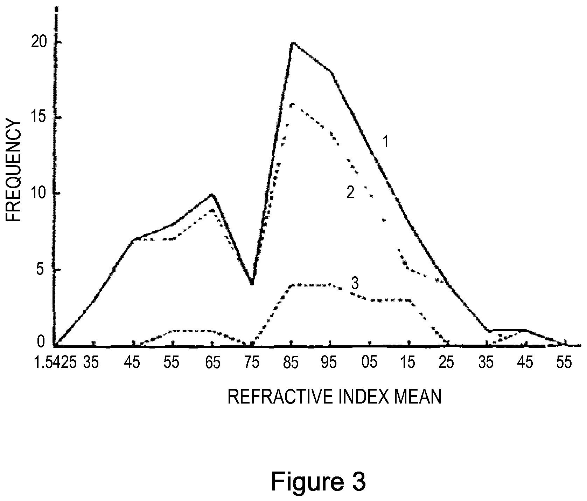

The graph in Fig. 3 illustrates the refractive index of hair, which can be found in a paper by M. D. Greenwell, A. Willner, Paul L. Kirk: Human Hair Studies: III. Refractive Index of Crown Hair, 31 Am. Inst. Crim. L. & Criminology 746 (1940-1941). Curve 1 is a composite line, curve 2 is a line representing the refractive index for Caucasian people, and curve 3 is a line representing the refractive index for non-Caucasian people. Thus, it can be seen that the refractive index of hair is between (approximately) 1.545 and 1.555, although there will be variation between individuals. For example the above paper also recognises that the refractive index of hair can depend on the sex of the subject, e.g. the refractive index of hair on a female is generally higher than the refractive index of hair on a male.

As is known, the first and second light guiding elements 6, 8 and the cutting element 10 together act as a waveguide for the light coupled from the one or more light sources 4 through the occurrence of total internal reflection, since the refractive index of air is lower than that of the optical waveguide. However, if an object that has a refractive index higher than the optical waveguide is put into contact with the cutting element 10, then the total internal reflection is 'frustrated' and light can couple from the optical waveguide into that object. Thus, in order for light to be coupled into a hair from the cutting element 10 part of the optical waveguide (to provide the cutting action according to the invention), the optical waveguide must have the same or a lower refractive index than hair at the point at which the hair contacts the cutting element 10. Thus, the optical waveguide must have the same or a lower refractive index than hair at least at the cutting face 20 portion of the cutting element. Preferably, the refractive index of the optical waveguide at the cutting face 20 is the same as that of hair since that provides the best coupling of light from the optical waveguide to the hair.

Thus, in some embodiments, the refractive index of the optical waveguide 10 at least at the cutting face 20 is equal to or lower than 1.56. More preferably the refractive index of the optical waveguide 10 at least at the cutting face 20 is equal to or lower than 1.55. Even more preferably, the refractive index of the optical waveguide 10 at least at the cutting face 20 is equal to or lower than 1.54, since this refractive index is below the refractive indices identified in Fig. 3.

In some embodiments, a lower bound for the refractive index of the optical waveguide 10 at the cutting face 20 can be 1.48, 1.51, 1.53 or 1.54.

A range of values from which the refractive index of the optical waveguide 10 is selected can be formed from any combination of the upper and lower refractive index bounds set out in the preceding paragraphs.

The optical waveguide/fibre 10 can be made from any suitable material or combination of materials. For example optical waveguides/fibres can be composed of or comprise silica, fluoride glass, phosphate glass, chalcogenide glass, crown glass (such as BK7) and/or crystals (such as sapphire or yttrium aluminium garnet (YAG)).

As noted above, including a tapered portion in the optical waveguide increases the numerical aperture of light propagating through the optical waveguide and, therefore, improves the ability of the cutting element to cut hair. It has been recognised, however, that light propagating from a first, proximal end of the optical waveguide 10 reduces in intensity as it propagates along the waveguide to the second, distal end of the waveguide. In some cases, the light intensity may reduce by such an extent that the ability of the cutting element 10 to cut hair at the distal end of the waveguide is reduced. Therefore, it has been recognised that increasing the intensity of light at the distal end of the optical waveguide 10 (such that the light intensity is sufficient to initiate the cutting or melting of hair) at both ends, and along the length, of the optical waveguide, can improve the effective cutting area of the cutting element 10 and, therefore, improve the effectiveness of the cutting element and the hair cutting device 2.

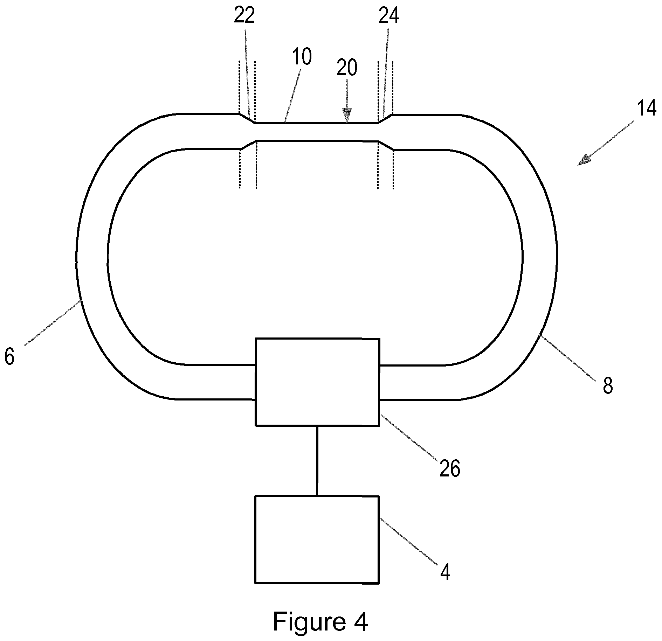

Fig. 4 shows a portion of a hair cutting device 2 constructed according to embodiments of the invention. Fig. 4 shows the cutting assembly 14 which comprises the cutting element 10, the first light guiding element 6 and the second light guiding element 8. The first light guiding element 6 includes a taper transition section, or portion, 22 which causes the diameter of the optical waveguide to reduce from a first diameter to a second diameter. Dashed lines indicate where the taper transition section 22 is located between the first light guiding element 6 and the cutting element 10. The second light guiding element 8 also includes a taper transition section 24 which causes the diameter of the optical waveguide to reduce from a first diameter to a second diameter. Dashed lines again indicate where the taper transition section 24 is located between the second light guiding element 8 and the cutting element 10. In some embodiments, the taper transition sections 22, 24 have the same taper ratios, such that the change in diameters of the optical waveguide in both sections is the same. Similarly, in some embodiments, the first diameter of the light guiding element 6 (i.e. the diameter of the optical waveguide on the light source side of the taper transition section 22) is the same as the diameter of the light guiding element 8 (i.e. the diameter of the optical waveguide on the light source side of the taper transition section 24), while in other embodiments, the diameters of the light guiding elements 6 and 8 may be different.

Thus, in general, the cutting assembly 14 comprises a cutting element 10 having a first end and a second end, the cutting element comprising an optical waveguide for receiving light from at the least one light source 4. The cutting assembly 14 further comprises a first light guiding element 6 configured to guide light from the at least one light source 4 into the first end of the cutting element 10, and a second light guiding element 8 configured to guide light from the at least one light source 4 into the second end of the cutting element 10. Each of the light guiding elements 6, 8 comprises a taper transition section 22, 24 in which a diameter of the light guiding element 6, 8 reduces from a first diameter to a second diameter.

In the embodiment shown in Fig. 4, the hair cutting device 2 includes a single light source 4, which is configured to generate light, for example laser light, at one or more particular wavelengths. In some embodiments, the cutting assembly 14 may further include a beam splitter 26 for dividing light from the at least one light source 4 into two paths, a first path along the first light guiding element 6 from the beam splitter 26 to the first end of the cutting element 10, and a second path along the second light guiding element 8 from the beam splitter 26 to the second end of the cutting element 10. While, in the embodiment of Fig. 4, a single light source 4 generates the light to be divided by the beam splitter 26, in other embodiments, two or more light sources may provide the light to be divided and delivered to the cutting element 10.

The beam splitter may be any known type of beam splitter suitable for dividing light, or a beam of light, from a source into two or more paths. In some embodiments, the beam splitter comprises a 50-50 coupler, which divides the incident light evenly into two paths. In other embodiments, a coupler or beam splitter having a different splitting ratio may be used. The beam splitter 26 may, in some embodiments, form part of the cutting assembly 14 (i.e. with the cutting element 10 and the first and second light guiding elements 6, 8). In other embodiments, the beam splitter may be located elsewhere in the hair cutting device 2 such that it does not form part of the cutting assembly 14.

Light from the single light source 4 is delivered along the first and second light guiding elements 6, 8 to the first and second ends respectively of the cutting element 10. In this way, the intensity of the light propagating into the cutting element 10 at the first end is the same as the intensity of the light propagating into the cutting element 10 at the second end. The length of the cutting element 10 may be selected such that the reduction in intensity of light as it reaches the central region (i.e. the region midway between the first and second ends) of the cutting element is negligible or small enough not to have a significant effect on the cutting ability/effectiveness of the device 2.

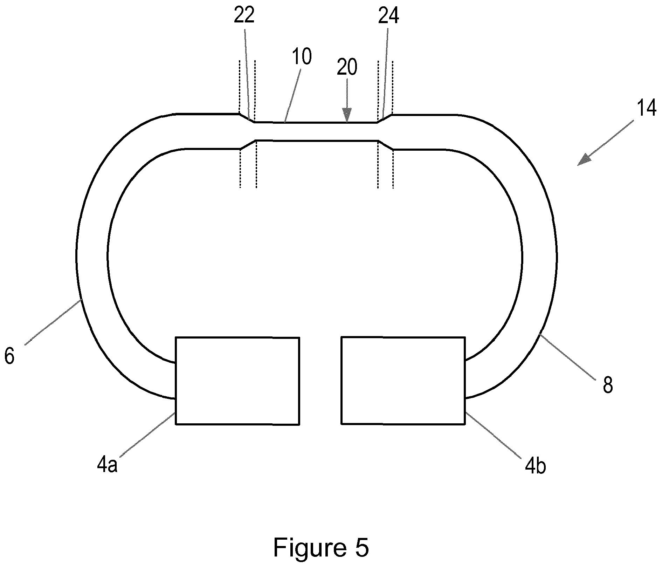

Fig. 5 shows a portion of a hair cutting device 2 constructed according to alternative embodiments of the invention. The cutting assembly 14 of the embodiment in Fig. 5 is identical to the cutting assembly shown in Fig. 4. In Fig. 5, however, the beam splitter 26 and the single light source 4 are replaced by a first light source 4a and a second light source 4b. In other words, the at least one light source 4 comprises a first light source 4a and a second light source 4b. The first light guiding element 6 is configured to guide light from the first light source 4a to the first end of the cutting element 10, and the second light guiding element 8 is configured to guide light from a second light source 4b to the second end of the cutting element 10. The first and second light sources 4a, 4b may be configured to generate light having the same wavelength, or light having two or more different wavelengths. In some embodiments, one or more beam splitters (such as beam splitter 26) may be incorporated into an arrangement having multiple light sources 4a, 4b such that light having different wavelengths may be delivered to both ends of the cutting element 10.

In any of the embodiments described herein, the first light guiding element 6 and/or the second light guiding element 8 may comprise a portion of the optical waveguide (e.g. the optical fibre). In other words, the first and second light guiding elements 6, 8 and the cutting element 10 may all form part of the same optical waveguide. At least one of the first light guiding element 6, the second light guiding element 8 and the cutting element 10 may comprise an optical fibre. Known methods of forming the taper transition sections of the optical waveguide may be used, such as heating and stretching the optical waveguide to form a taper transition section having the required ratio and length.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A cutting assembly for use in a hair cutting device, the cutting assembly comprising:
a cutting element having a first end and a second end, the cutting element comprising an optical waveguide for receiving light from at least one light source;
a first light guiding element configured to guide light from the at least one light source into the first end of the cutting element; and
a second light guiding element configured to guide light from the at least one light source into the second end of the cutting element;
wherein each of the light guiding elements comprises a taper transition section in which a diameter of the light guiding element reduces from a first diameter to a second diameter.

2. A cutting assembly according to claim 1, further comprising:
a beam splitter for dividing light from the at least one light source into two paths, a first path along the first light guiding element from the beam splitter to the first end of the cutting element, and a second path along the second light guiding element from the beam splitter to the second end of the cutting element.

3. A cutting assembly according to claim 2, wherein the beam splitter comprises a 50-50 coupler.

4. A cutting assembly according to claim 1, wherein the first light guiding element is configured to guide light from a first light source to the first end of the cutting element, and wherein the second light guiding element is configured to guide light from a second light source to the second end of the cutting element.

5. A cutting assembly according to any of the preceding claims, wherein the first light guiding element and/or the second light guiding element comprises a portion of the optical waveguide.

6. A cutting assembly according to any of the preceding claims, wherein at least one of the first light guiding element, the second light guiding element and the cutting element comprises an optical fibre.

7. A hair cutting device for cutting hair on a body of a subject, the hair cutting device comprising:
at least one light source for generating laser light at one or more specific wavelengths corresponding to wavelengths absorbed by one or more chromophores in hair; and
a cutting assembly coupled to the at least one light source, the cutting assembly comprising:
a cutting element having a first end and a second end, the cutting element comprising an optical waveguide for receiving light from the at least one light source;
a first light guiding element configured to guide light from the at least one light source into the first end of the cutting element; and
a second light guiding element configured to guide light from the at least one light source into the second end of the cutting element;
wherein each of the light guiding elements comprises a taper transition section in which a diameter of the light guiding element reduces from a first diameter to a second diameter.

8. A hair cutting device according to claim 7, wherein the cutting assembly further comprises:
a beam splitter for splitting light from the at least one light source into two paths, a first path along the first light guiding element from the beam splitter to the first end of the cutting element, and a second path along the second light guiding element from the beam splitter to the second end of the cutting element.

9. A hair cutting device according to claim 8, wherein the beam splitter comprises a 50-50 coupler.

10. A hair cutting device according to any of claims 7 to 9, wherein the at least one light source comprise a single light source.

11. A hair cutting device according to claim 7, wherein the at least one light source comprises a first light source and a second light source;
wherein light from the first light source is guided along the first light guiding element to the first end of the cutting element; and
wherein light from the second light source is guided along the second light guiding element to the second end of the cutting element.

12. A hair cutting device according to any of claims 7 to 11, wherein the first light guiding element and/or the second light guiding element comprises a portion of the optical waveguide.

13. A hair cutting device according to any of claims 7 to 12, wherein at least one of the first light guiding element, the second light guiding element and the cutting element comprises an optical fibre.

## Patentansprüche

1. Schneidanordnung zur Verwendung in einer Haarschneidevorrichtung, wobei die Schneidanordnung umfasst:
ein Schneidelement, das ein erstes Ende und ein zweites Ende aufweist, wobei das Schneidelement einen Lichtwellenleiter zum Empfangen von Licht von mindestens einer Lichtquelle umfasst;
ein erstes Lichtleitelement, das konfiguriert ist, um Licht von der mindestens einen Lichtquelle in das erste Ende des Schneidelements zu leiten; und
ein zweites Lichtleitelement, das konfiguriert ist, um Licht von der mindestens einen Lichtquelle in das zweite Ende des Schneidelements zu leiten;
wobei jedes der Lichtleitelemente einen Verjüngungsübergangsabschnitt umfasst, in dem sich ein Durchmesser des Lichtleitelements von einem ersten Durchmesser zu einem zweiten Durchmesser reduziert.

2. Schneidanordnung nach Anspruch 1, weiter umfassend:
einen Strahlenteiler zum Teilen von Licht aus der mindestens einen Lichtquelle in zwei Wege, einen ersten Weg entlang des ersten Lichtleitelements von dem Strahlenteiler zu dem ersten Ende des Schneidelements, und einen zweiten Weg entlang des zweiten Lichtleitelements von dem Strahlenteiler zu dem zweiten Ende des Schneidelements.

3. Schneidanordnung nach Anspruch 2, wobei der Strahlenteiler einen 50-50-Koppler umfasst.

4. Schneidanordnung nach Anspruch 1, wobei das erste Lichtleitelement konfiguriert ist, um Licht von einer ersten Lichtquelle zu dem ersten Ende des Schneidelements zu leiten, und wobei das zweite Lichtleitelement konfiguriert ist, um Licht von einer zweiten Lichtquelle zu dem zweiten Ende des Schneidelements zu leiten.

5. Schneidanordnung nach einem der vorstehenden Ansprüche, wobei das erste Lichtleitelement und/oder das zweite Lichtleitelement einen Abschnitt des Lichtwellenleiters umfasst.

6. Schneidanordnung nach einem der vorstehenden Ansprüche, wobei mindestens eines des ersten Lichtleitelements, des zweiten Lichtleitelements und des Schneidelements eine Lichtleitfaser umfasst.

7. Haarschneidevorrichtung zum Schneiden von Haar auf einem Körper eines Subjekts, wobei die Haarschneidevorrichtung umfasst:
mindestens eine Lichtquelle zum Erzeugen von Laserlicht mit einer oder mehreren spezifischen Wellenlängen, die Wellenlängen entsprechen, die von einem oder mehreren Chromophoren in Haar absorbiert werden; und
eine Schneidanordnung, die mit der mindestens einen Lichtquelle gekoppelt ist, wobei die Schneidanordnung umfasst:
ein Schneidelement, das ein erstes Ende und ein zweites Ende aufweist, wobei das Schneidelement einen Lichtwellenleiter zum Empfangen von Licht von der mindestens einen Lichtquelle umfasst;
ein erstes Lichtleitelement, das konfiguriert ist, um Licht von der mindestens einen Lichtquelle in das erste Ende des Schneidelements zu leiten; und
ein zweites Lichtleitelement, das konfiguriert ist, um Licht von der mindestens einen Lichtquelle in das zweite Ende des Schneidelements zu leiten;
wobei jedes der Lichtleitelemente einen Verjüngungsübergangsabschnitt umfasst, in dem sich ein Durchmesser des Lichtleitelements von einem ersten Durchmesser zu einem zweiten Durchmesser reduziert.

8. Haarschneidevorrichtung nach Anspruch 7, wobei die Schneidanordnung weiter umfasst:
einen Strahlenteiler zum Teilen von Licht von der mindestens einen Lichtquelle in zwei Wege, einen ersten Weg entlang des ersten Lichtleitelements von dem Strahlenteiler zu dem ersten Ende des Schneidelements, und einen zweiten Weg entlang des zweiten Lichtleitelements von dem Strahlenteiler zu dem zweiten Ende des Schneidelements.

9. Haarschneidevorrichtung nach Anspruch 8, wobei der Strahlenteiler einen 50-50-Koppler umfasst.

10. Haarschneidevorrichtung nach einem der Ansprüche 7 bis 9, wobei die mindestens eine Lichtquelle eine einzige Lichtquelle umfasst.

11. Haarschneidevorrichtung nach Anspruch 7, wobei die mindestens eine Lichtquelle eine erste Lichtquelle und eine zweite Lichtquelle umfasst;
wobei Licht von der ersten Lichtquelle entlang des ersten Lichtleitelements zu dem ersten Ende des Schneidelements geleitet wird; und
wobei Licht von der zweiten Lichtquelle entlang des zweiten Lichtleitelements zu dem zweiten Ende des Schneidelements geleitet wird.

12. Haarschneidevorrichtung nach einem der Ansprüche 7 bis 11, wobei das erste Lichtleitelement und/oder das zweite Lichtleitelement einen Abschnitt des Lichtwellenleiters umfasst.

13. Haarschneidevorrichtung nach einem der Ansprüche 7 bis 12, wobei mindestens eines des ersten Lichtleitelements, des zweiten Lichtleitelements und des Schneidelements eine Lichtleitfaser umfasst.

## Revendications

1. Ensemble de coupe pour l'utilisation dans un dispositif de coupe de poils, l'ensemble de coupe comprenant :
un élément de coupe présentant une première extrémité et une seconde extrémité, l'élément de coupe comprenant un guide d'onde optique pour la réception de la lumière d'au moins une source de lumière ;
un premier élément de guidage de lumière configuré pour guider de la lumière de l'au moins une source de lumière dans la première extrémité de l'élément de coupe ; et
un second élément de guidage de lumière configuré pour guider de la lumière de l'au moins une source de lumière dans la seconde extrémité de l'élément de coupe ;
dans lequel chacun des éléments de guidage de lumière comprend une section de transition conique dans laquelle un diamètre de l'élément de guidage de lumière se réduit d'un premier diamètre à un second diamètre.

2. Ensemble de coupe selon la revendication 1, comprenant en outre :
un séparateur de faisceau pour la division de lumière de l'au moins une source de lumière en deux trajets, un premier trajet le long du premier élément de guidage de lumière du séparateur de faisceau à la première extrémité de l'élément de coupe, et un second trajet le long du second élément de guidage de lumière du séparateur de faisceau à la seconde extrémité de l'élément de coupe.

3. Ensemble de coupe selon la revendication 2, dans lequel le séparateur de faisceau comprend un coupleur 50-50.

4. Ensemble de coupe selon la revendication 1, dans lequel le premier élément de guidage de lumière est configuré pour guider de la lumière d'une première source de lumière à la première extrémité de l'élément de coupe, et dans lequel le second élément de guidage de lumière est configuré pour guider de la lumière d'une seconde source de lumière à la seconde extrémité de l'élément de coupe.

5. Ensemble de coupe selon l'une quelconque des revendications précédentes, dans lequel le premier élément de guidage de lumière et/ou le second élément de guidage de lumière comprend une portion du guide d'onde optique.

6. Ensemble de coupe selon l'une quelconque des revendications précédentes, dans lequel au moins un du premier élément de guidage de lumière, du second élément de guidage de lumière et de l'élément de coupe comprend une fibre optique.

7. Ensemble de coupe de poils pour la coupe de poils sur un corps d'un sujet, le dispositif de coupe de poils comprenant :
au moins une source de lumière pour la génération de lumière laser à une ou plusieurs longueurs d'onde spécifiques correspondant aux longueurs d'onde absorbées par un ou plusieurs chromophores dans les poils ; et
un ensemble de coupe couplé à l'au moins une source de lumière, l'ensemble de coupe comprenant :
un élément de coupe présentant une première extrémité et une seconde extrémité, l'élément de coupe comprenant un guide d'onde optique pour la réception de lumière de l'au moins une source de lumière ;
un premier élément de guidage de lumière configuré pour guider de la lumière de l'au moins une source de lumière dans la première extrémité de l'élément de coupe ; et
un second élément de guidage de lumière configuré pour guider de la lumière de l'au moins une source de lumière dans la seconde extrémité de l'élément de coupe ;
dans lequel chacun des éléments de guidage de lumière comprend une section de transition conique dans laquelle un diamètre de l'élément de guidage de lumière se réduit d'un premier diamètre à un second diamètre.

8. Dispositif de coupe de poils selon la revendication 7, dans lequel l'ensemble de coupe comprend en outre :
un séparateur de faisceau pour la séparation de lumière de l'au moins une source de lumière en deux trajets, un premier trajet le long du premier élément de guidage du séparateur de faisceau à la première extrémité de l'élément de coupe, et un second trajet le long du second élément de guidage de lumière du séparateur de faisceau à la seconde extrémité de l'élément de coupe.

9. Dispositif de coupe de poils selon la revendication 8, dans lequel le séparateur de faisceau comprend un coupleur 50-50.

10. Dispositif de coupe de poils selon l'une quelconque des revendications 7 à 9, dans lequel l'au moins une source de lumière comprend une source de lumière seule.

11. Dispositif de coupe de poils selon la revendication 7, dans lequel l'au moins une source de lumière comprend une première source de lumière et une seconde source de lumière ;
dans lequel de la lumière de la première source de lumière est guidée le long du premier élément de guidage de lumière à la première extrémité de l'élément de coupe ; et
dans lequel de la lumière de la seconde source de lumière est guidée le long du second élément de guidage de lumière à la seconde extrémité de l'élément de coupe.

12. Dispositif de coupe de poils selon l'une quelconque des revendications 7, à 11, dans lequel le premier élément de guidage de lumière et/ou le second élément de guidage de lumière comprend une portion du guide d'onde optique.

13. Dispositif de coupe de poils selon l'une quelconque des revendications 7, à 12, dans lequel au moins un du premier élément de guidage de lumière, du second élément de guidage de lumière et de l'élément de coupe comprend une fibre optique.
